(19) Europäisches Patentamt
European Patent Office
Office européen des brevets

(11) Veröffentlichungsnummer : **0 046 871 B1**

(12) # EUROPÄISCHE PATENTSCHRIFT

(45) Veröffentlichungstag der Patentschrift : **07.12.83**

(51) Int. Cl.³ : **G 03 C 1/34**, G 03 C 1/37, C 07 D249/12, C 07 D249/14

(21) Anmeldenummer : **81105776.9**

(22) Anmeldetag : **22.07.81**

(54) **Fotografisches Material, Herstellungsverfahren, Verfahren zur Herstellung fotografischer Bilder sowie neue Triazole.**

(30) Priorität : **02.09.80 DE 3032945**

(43) Veröffentlichungstag der Anmeldung : **10.03.82 Patentblatt 82/10**

(45) Bekanntmachung des Hinweises auf die Patenterteilung : **07.12.83 Patentblatt 83/49**

(84) Benannte Vertragsstaaten : **BE CH DE FR GB LI**

(56) Entgegenhaltungen :
**DE-A- 2 711 942**
**DE-A- 2 948 937**
**DE-C- 2 304 321**
**GB-A- 741 228**

(73) Patentinhaber : **AGFA-GEVAERT Aktiengesellschaft**
**D-5090 Leverkusen 1 (DE)**

(72) Erfinder : **von König, Anita, Dr.**
**Schönwasserstrasse 230**
**D-4150 Krefeld (DE)**
Erfinder : **Liebe, Werner, Dr.**
**Am Thelenhof 28**
**D-5090 Leverkusen (DE)**
Erfinder : **Saleck, Wilhelm, Dr.**
**Im Birkelshof 3**
**D-5060 Bergisch Gladbach 2 (DE)**

**EP 0 046 871 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Fotografisches Material, Herstellungsverfahren, Verfahren zur Herstellung fotografischer Bilder sowie neue Triazole

Die Erfindung betrifft ein fotografisches Material mit einem Stabilisierungsmittel, ein Verfahren zur Herstellung dieses Materials, ein Verfahren zur Herstellung fotografischer Bilder sowie neue Triazole. Die Erfindung betrifft insbesondere ein fotografisches Material mit mindestens einer Silberhalogenidemulsionsschicht, welches durch den Zusatz der erfindungsgemäßen Stabilisierungsmittel gegen die Bildung von Schleier, insbesondere Farbschleier, stabilisiert ist.

Materialien mit lichtempfindlichen Silberhalogenidemulsionen, insbesondere chemisch sensibilisierte, neigen bekanntlich zur Bildung von Schleiern, hervorgerufen durch Keime, die ohne Belichtung entwickelbar sind. Diese Schleierbildung tritt insbesondere auf bei zu langer Lagerung, besonders bei erhöhter Temperatur und Luftfeuchtigkeit, bei zu langer Entwicklung oder Entwicklung bei zu hohen Temperaturen, durch bestimmte Zusätze und bei stark gereiften Emulsionen.

Bei der Schnellverarbeitung fotografischer Materialien bei Temperaturen von über 30 °C und Entwicklungs-, Fixier- bzw. Bleichfixierzeiten von unter 6 Minuten in den einzelnen Bädern tritt häufig eine stärkere Verschleierung auf als bei der üblichen langsameren Entwicklung bei 20 °C.

Es ist bekannt, fotografischen Silberhalogenidemulsionen zur Verminderung dieser Schleierbildung sogenannte Antischleiermittel oder Stabilisierungsmittel zuzusetzen. Stabilisierende Wirkung besitzen z. B. heterocyclische Verbindungen, die Schwefel, beispielsweise in Form einer Mercaptogruppe, enthalten. Beispielsweise sei hingewiesen auf die deutschen Auslegeschriften 1 183 371, 1 189 380, 1 597 503, 1 797 027 und auf die deutschen Offenlegungsschriften 1 522 363, 2 042 533, 2 130 031, 2 308 530 und 2 943 673.

Diesen Stabilisierungsmitteln haftet jedoch als Nachteil an, daß sie in wirksamen Konzentrationen unter Umständen die Empfindlichkeit der stabilisierten Emulsion herabsetzen, wodurch deren Anwendbarkeit beeinträchtigt wird. Auch die Gradation der Emulsion kann durch diese Stabilisatoren ungünstig beeinflußt werden.

Bei der Verarbeitung des stabilisierten fotografischen Materials können Stabilisierungsmittel, wenn auch in sehr kleiner Menge, in den Entwickler und/oder in das Bleichfixierbad eingeschleppt werden. Bei Verarbeitung großer Mengen dieser Materialien reichern sich die Verbindungen im Entwickler und/oder Bleichfixierbad an. Ersteres hat den Nachteil, daß die Entwicklungsgeschwindigkeit abnimmt oder, im Falle von lichtempfindlichen farbfotografischen Mehrschichten-Materialien, das Farbgleichgewicht verloren geht. Letzteres hat den Nachteil, daß die Bleichgeschwindigkeit abnimmt, wodurch bei kurzen Bleichfixierzeiten, wie sie bei der Schnellverarbeitung von fotografischen Materialien notwendig sind, ein Schleier durch nicht entferntes Silber entsteht.

Beide Nachteile sind besonders ausgeprägt, wenn ein zurückgewonnener und regenerierter Entwickler und/oder ein zurückgewonnenes oder regeneriertes Bleichfixierbad verwendet wird.

Der Erfindung lag die Aufgabe zugrunde, Stabilisierungsmittel aufzufinden, die fotografische Materialien gegen Schleier und Farbschleier, insbesondere bei Verarbeitung bei höheren Temperaturen, stabilisieren und die auch bei Verwendung von zurückgewonnenen und regenerierten Verarbeitungsbädern weder die Entwicklung noch die Bleichung verzögern.

Eine weitere Aufgabe ist die Bereitstellung von fotografischen Materialien mit mindestens einer Silberhalogenidemulsionsschicht, die mit diesen Verbindungen stabilisiert sind. Weitere Aufgaben ergeben sich aus der Beschreibung.

Es wurde nun gefunden :

1. Ein fotografisches Material aus einem Schichtträger und wenigstens einer darauf aufgetragenen lichtempfindlichen Silberhalogenidemulsionsschicht und gegebenenfalls weiteren Schichten, wobei in wenigstens einer Schicht eine Verbindung der folgenden Formel (I) enthalten ist :

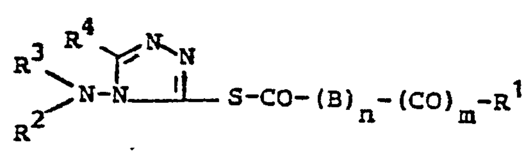

(I)

worin bedeuten

$R^1$ einen Alkyl-, Cycloalkyl-, Aryl- oder Aralkylrest oder einen substituierten über ein —S-Atom gebundenen Triazolrest,

$R^2$ H, Acyl, Alkyl oder —$COOR^5$,

$R^3$ H, Acyl oder —$COOR^5$,

$R^4$ H oder Alkyl,

$R^5$ Alkyl, Cycloalkyl, Aryl, Aralkyl

n, m gleich oder verschieden, 0 oder 1,

B —O—, Alkylen, Arylen, Cycloalkylen oder Aralkylen, wobei diese organischen Reste an ein oder zwei Stellen ein Sauerstoffatom tragen können

und wobei die angegebenen Substituenten ihrerseits mit Substituenten substituiert sein können, die für

Substituenten auf dem fotografischen Gebiet üblich sind.

2. Verfahren zur Herstellung lichtempfindlicher silberhalogenidhaltiger fotografischer Materialien durch Fällen des Silberhalogenids, Reifung und Auftragen auf einen Schichtträger, dadurch gekennzeichnet, daß der Silberhalogenidemulsion vor dem Auftragen eine Verbindung der Formel (I) zugesetzt wird.

3. Ein Verfahren zur Herstellung fotografischer Bilder durch bildmäßige Belichtung und Entwicklung des erfindungsgemäßen Materials.

4. Neue Verbindungen der angegebenen Formel (I).

$R^1$ ist bevorzugt ein Alkylrest mit 1 bis 6 C-Atomen, insbesondere Methyl, Ethyl, Isopropyl, Butyl oder ein Cycloalkylrest mit 5 oder 6 C-Atomen, insbesondere Cyclohexyl oder ein Phenylrest oder ein Benzylrest oder ein Rest der Formel

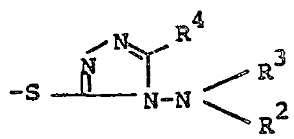

in welcher

$R^2$-$R^4$ die oben angegebene Bedeutung haben.

$R^2$ ist bevorzugt H, Acetyl, Alkyl mit 1 bis 4 C-Atomen oder $COOR^5$.

$R^3$ ist bevorzugt H oder ein Acylrest. Ein besonders bevorzugter Acylrest ist der Acetylrest.

$R^4$ bedeutet bevorzugt H oder einen Alkylrest mit 1 bis 4 C-Atomen, insbesondere Methyl oder Ethyl.

$R^5$ bedeutet vorzugsweise Alkyl mit 1 bis 6 C-Atomen oder Cycloalkyl mit 5 oder 6 C-Atomen, insbesondere Cyclohexyl oder Phenyl oder Benzyl.

Das Bindeglied B ist bevorzugt —O— oder ein Alkylenrest, insbesondere mit 1 bis 6 C-Atomen, ein Arylenrest, insbesondere ein Phenylenrest, ein Cycloalkylenrest, insbesondere mit 5 oder 6 C-Atomen oder ein Aralkylenrest, insbesondere ein Rest der Formel

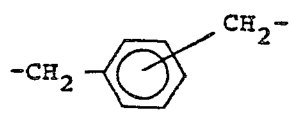

Wenn B ein organischer Rest ist, kann dieser entweder nur an einer aber auch an zwei Stellen ein Sauerstoffatom tragen, bevorzugt endständig.

Spezielle Beispiele für B sind :

—O—

$—O—[CH_2]_n—O—$ mit n = 1-4

$—[CH_2]_n—$ mit n = 1-4

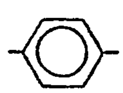 oder 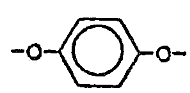

In einer bevorzugten Ausführungsform ist das Bindeglied dann ein organischer Rest, wenn m = 1. Die angegebenen Substituenten können gegebenenfalls ihrerseits wieder substituiert sein. Hierbei handelt es sich um Substituenten, die für Stabilisatoren auf dem fotografischen Gebiet üblich sind. Beispielsweise kann Halogen, Alkoxy, Cyan, Carboxy, $COOR^5$ verwendet werden.

Besonders geeignete Verbindungen der Formel I sind in folgenden Tabelle 1 angegeben :

(Siehe Tabelle 1, Seite 4 ff.)

| Nr. | $-(B)_n-(CO)_m-R^1$ | $R^2$ | $R^3$ | $R^4$ |
|---|---|---|---|---|
| 1.1 | $-CH_3$ | $-CO-CH_3$ | H | H |
| 1.2 | $-O-C_4H_9-$ | $-CO-O-C_4H_9$-n | H | H |
| 1.3 | $-O-C_2H_5$ | $-CO-O-C_2H_5$ | $-CO-CH_3$ | H |
| 1.4 | $-O-C_4H_9$-n | $-CO-O-C_4H_9$-n | $-CO-CH_3$ | H |
| 1.5 | $-O-C_4H_9$-iso | $-CO-O-C_4H_9$-iso | $-CO-CH_3$ | H |
| 1.6 | $-O-C_2H_5$ | $-CO-O-C_2H_5$ | $-COO-C_2H_5$ | H |
| 1.7 | 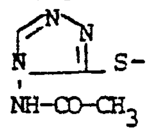 | $-CO-CH_3$ | H | H |
| 1.8 | $-CH_3$ | $-CO-CH_3$ | H | $CH_3$ |
| 1.9 | $-O-C_2H_5$ | $-CO-O-C_2H_5$ | $-CO-CH_3$ | $CH_3$ |
| 1.10 | $-O-C_4H_9$-n | $-CO-O-C_4H_9$-n | $-CO-CH_3$ | $CH_3$ |
| 1.11 | $-O-C_4H_9$-n | H | H | $CH_3$ |
| 1.12 | 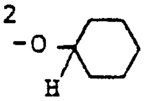 | 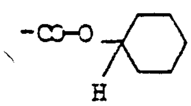 | H | $CH_3$ |
| 1.13 | $-O-CH(CH_3)_2$ | $-CO-O-CH(CH_3)_2$ | $-CO-CH_3$ | $CH_3$ |
| 1.14 | $-O-C_4H_9$-iso | $-CO-O-C_4H_9$-iso | $-CO-CH_3$ | $CH_3$ |
| 1.15 | $-O-C_4H_9$-n | $-CO-O-C_4H_9$-n | H | $CH_3$ |
| 1.16 | 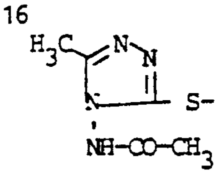 | $-CO-CH_3$ | H | $CH_3$ |
| 1.17 | 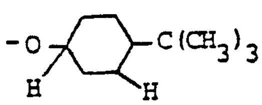 | 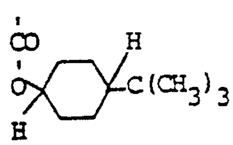 | H | $CH_3$ |
| 1.18 | 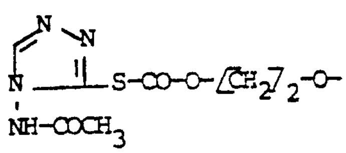 | $-COCH_3$ | H | H |
| 1.19 | 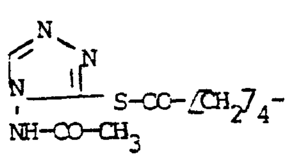 | $-CO-CH_3$ | H | H |
| 1.20 | 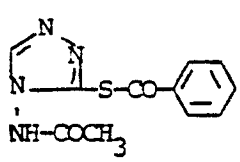 | $-COCH_3$ | H | $CH_3$ |
| 1.21 | 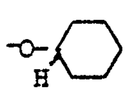 | 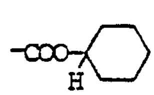 | $-COCH_3$ | H |

(Fortsetzung)

| Nr. | $-(B)_n-(CO)_m-R^1$ | $R^2$ | $R^3$ | $R^4$ |
|---|---|---|---|---|
| 1.22 | $-O-C_4H_9$-iso | $-COO-C_4H_9$-iso | H | H |
| 1.23 | $-O-C_2H_5$ | H | H | H |
| 1.24 | 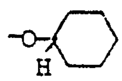 | 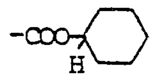 | $-COCH_3$ | $CH_3$ |
| 1.25 | $-CH_3$ | $-COCH_3$ | H | $C_2H_5$ |
| 1.26 | $-O-C_2H_5$ | $-COO-C_2H_5$ | H | $C_2H_5$ |
| 1.27 | 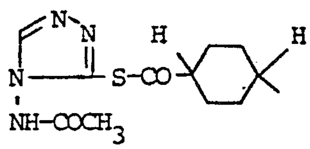 | $CO-CH_3$ | H | H |
| 1.28 | 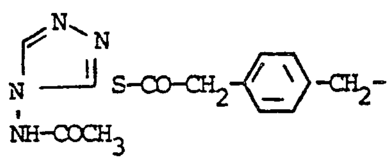 | $CO-CH_3$ | H | H |

Die Verbindungen der allgemeinen Formel (I) können nach bekannten Verfahren hergestellt werden. Die Verbindung 1.1 wird aus den zugrundeliegendem 3-Mercapto-4-amino-1,2,4-triazol und die Verbindung 1.8 wird aus Thiocarbohydrazid durch Ringschluß und durch Acylierung mit Essigsäureanhydrid im Eisessig hergestellt. Die Verbindungen 1.2 bis 1.6, 1.9 bis 1.15 und 1.17 bis 1.28 werden durch Umsatz der zugrundeliegenden 3-Mercapto-4-amino-1,2,4-triazole mit Chlorameisensäureestern, Pyrokohlensäureestern oder Säurechloriden vorzugsweise in Verdünnungsmitteln wie in DE-C-1 189 380 und DE-A-2 042 533 beschrieben hergestellt.

Die Verbindungen 1.7 und 1.16 werden in bekannter Weise durch Umsatz mit Phosgen in einem Verdünnungsmittel in Gegenwart von Alkali wie in DE-C-1 797 027 beschrieben hergestellt. Die Herstellung einiger Verbindungen wird im folgenden im einzelnen beschrieben ; die übrigen werden in analoger Weise erhalten.

Herstellung der Verbindung 1.1

Eine Suspension von 23,2 g (0,2 Mol) 3-Mercapto-4-amino-1,2,4-triazol werden unter Rühren in 70 ml Eisessig zum Sieden erhitzt. In die siedende Lösung werden 56,7 ml Essigsäureanhydrid zugetropft und 30 Minuten bei Siedetemperatur gerührt. Nach dem Abkühlen wird abgesaugt und das Reaktionsprodukt durch mehrmaliges Umfällen aus Aceton und Petrolether gereinigt. Ausbeute 10,4 g ; Zersetzungspunkt 128 bis 130 °C.

Herstellung der Verbindung 1.4

Zu einer Suspension von 15,8 g (0,1 Mol) 3-Mercapto-4-acetylamino-1,2,4-triazol in 300 ml Aceton werden unter Rühren 40 ml 5 n Natronlauge zugesetzt und bei 0 bis 5 °C langsam 27,1 ml Chlorameisensäure-n-butylester gelöst in 100 ml getrockneten Aceton zugetropft. Nach 4-stündigem Rühren bei Raumtemperatur wird das ausgefallene Salz abgesaugt und die Lösung im Vakuum eingeengt. Der ölige Rückstand wird in Ether gelöst, gewaschen und nach Verrühren mit Aluminiumoxid und Aktivkohle und Trocknen wird der Ether abdestilliert. Die ölige Substanz wird durch mehrmaliges Lösen in Chloroform und in Essigsäureethylester und Behandlung der Lösungen mit Aktivkohle und Aluminiumoxid gereinigt. Ausbeute 7,5 g eines zähflüssigen Öls.

Die Analyse ergab :

| | % ber. | % gef. |
|---|---|---|
| C | 47,0 | 47,2 ; 47,1 |
| H | 6,17 | 6,2 ; 6,3 |
| N | 15,62 | 15,7 |
| O | 22,31 | 22,1 |
| S | 8,94 | 9,2 |

Herstellung der Verbindung 1.8

Eine Suspension von 21,23 g (0,2 Mol) Thiocarbohydrazid wird unter Ausschuß von Feuchtigkeit und Rühren in 100 ml Eisessig zum Sieden erhitzt.

In die siedende Lösung werden 85.05 ml (0,9 Mol) Essigsäureanhydrid zugetropft. Das Reaktionsgemisch wird 5 Stunden unter Rühren gekocht und anschließend im Wasserstrahl-Vakuum bis zur Trockene eingeengt. Das Produkt wird nach dem Abkühlen auf 25 bis 30 °C mit 30 ml Aceton versetzt und kräftig gerührt, wobei das Produkt kristallin wird. Nach dem Absaugen wird das Produkt mit $H_2O$ verrührt, scharf abgesaugt und anschließend zweimal mit Aceton verrührt. Anschließend wird das Produkt im Vakuum-Trockenschrank über $P_2O_5$ bei Raumtemperatur getrocknet. Ausbeute 16,5 g ; Zersetzungspunkt 176 bis 180 °C.

Es wurden folgende NMR $^{13}$C-Shifts gefunden :

| NMR $^{13}$C-Shifts | (ppm, rel. zu TMS = O) |
|---|---|
| C-3 | 166.707 |
| $CH_3$ an C-5 | 9.845 |
| C-5 | 149.818 |
| CO von NHAc | 168.423 |
| $CH_3$ von NHAc | 20.304 |
| CO von SAc | 167.088 |
| $CH_3$ von SAc | 24.173 |

Herstellung der Verbindung 1.9

Zu einer Suspension von 17,2 g (0,1 Mol) 3-Mercapto-4-acetylamino-5-methyl-1,2,4-triazol in 200 ml Aceton werden unter Rühren 40 ml 5 n Natronlauge zugesetzt und bei 0 bis 5 °C 21 ml Chlorameisensäureethylester gelöst in 100 ml getrocknetem Aceton zugetropft. Nach 4-stündigem Rühren bei Raumtemperatur wird das ausgefallene Salz abfiltriert und die Lösung im Vakuum eingeengt. Der Rückstand wird in 100 ml Aceton gelöst und die Lösung mit Aktivkohle behandelt. Nach Abdestillieren des Acetons im Vakuum wird das Produkt mehrmals mit Petrolether und Ether verrührt. Ausbeute 7,8 g ; Zersetzungspunkt 98 bis 99 °C.

Herstellung der Verbindung 1.10

Zu einer Lösung von 21,4 g (0,1 Mol) der Verbindung 1.8 in 100 ml Aceton werden unter Rühren 60 ml 5 n Natronlauge und bei 0 bis 5 °C 27,1 ml Chlorameisensäure-n-butylester gelöst in 100 ml getrocknetem Aceton zugetropft. Nach 3-stündigem Rühren bei Raumtemperatur wird das ausgefallene Salz abfiltriert und die Lösung im Vakuum eingeengt. Der Rückstand wird in Ether gelöst, gewaschen und nach Verrühren mit Aluminiumoxid und Aktivkohle und Trocknen wird der Ether abdestilliert. Durch mehrmaliges Lösen in Aceton und Behandlung mit Aktivkohle und Aluminiumoxid wird das Öl gereinigt. Ausbeute 21,5 g eines zähflüssigen Öls.

Die Analyse ergab :

| | % ber. | % gef. |
|---|---|---|
| C | 48,37 | 48,3 ; 48,4 |
| H | 6,50 | 6,2 ; 6,3 |
| N | 15,04 | 15,6 ; 15,6 |
| O | 21,48 | 21,6 |
| S | 8,61 | 8,7 |

Die erreichbare Empfindlichkeit eines fotografischen Materials mit den erfindungsgemäßen Stabilisatoren ist sehr hoch und wird während der Lagerung nicht vermindert. Die für den Fachmann sehr

wichtige Stabilität eines fotografischen Materials wird bei der Anwendung der erfindungsgemäßen Verbindungen in hervorragender Weise erreicht. Der sonst bei der Entwicklung im allgemeinen auftretende Schleier wird unterdrückt.

Es ist günstig, die erfindungsgemäßen Verbindungen in Form von Lösungen zuzusetzen. Geeignet als Lösungsmittel sind beispielsweise niedere Alkohole, Tetrahydrofuran oder Aceton.

Die Emulsionen können in Kombination mit den erfindungsgemäßen weitere Stabilisatoren enthalten. Als Stabilisatoren sind ferner geeignet Azaindene, vorzugsweise Tetra- oder Pentaazaindene, insbesondere solche, die mit Hydroxyl- oder Aminogruppen substituiert sind. Derartige Verbindungen sind in dem Artikel von Birr, Z. wiss. Phot. 47, 2-58 (1952) beschrieben. Weitere geeignete Stabilisatoren sind u. a. heterocyclische Mercaptoverbindungen, z. B. Phenylmercaptotetrazol, quaternäre Benzthiazolderivate, Benzotriazol und ähnliche.

Besonders bevorzugte zusätzlich verwendbare Stabilisatoren vom Azainden-Typ sind insbesondere die in Tabelle 2 aufgeführten :

Tabelle 2

| Nr. | Verbindung |
|---|---|
| 2.1 | 4-Hydroxy-6-methyl-1,3,3a,7-tetraazainden, |
| 2.2 | 4-Hydroxy-5-carboxy-1,3-3a,7-tetraazainden, |
| 2.3 | 4-Hydroxy-5-carbethoxy-1,3-3a,7-tetraazainden, |
| 2.4 | 2-β-Hydroxyethyl-4-hydroxy-6-methyl-1,3,3a,7-tetraazainden, |
| 2.5 | 2-Methyl-4-hydroxy-1,3,3a,7-tetraazainden, |
| 2.6 | 4-Hydroxy-6-methyl-1,2,3,3a,7-pentaazainden, |

Die erfindungsgemäßen Stabilisierungsmittel können den lichtempfindlichen Silberhalogenidemulsionen vor der chemischen Reifung, zur chemischen Reifung oder nach der chemischen Reifung zugesetzt werden. In einer bevorzugten Ausführungsform werden sie nach der chemischen Reifung zur fertigen Gießlösung zugesetzt.

Die erfindungsgemäßen Stabilisierungsmittel werden vorzugsweise den lichtempfindlichen Silberhalogenidemulsionen vor oder nach der chemischen Reifung zugesetzt. Selbstverständlich kann man die Stabilisierungsmittel auch anderen fotografischen Schichten zusetzen. Die Konzentration der Stabilisierungsmittel in der Emulsion kann innerhalb weiter Grenzen schwanken. Sie hängt von der Art der Emulsion und dem gewünschten Effekt ab. Im allgemeinen werden mit Mengen von 5 mg bis 500 mg, insbesondere von 10 bis 200 mg pro Mol Silberhalogenid, die gewünschten Effekte erreicht.

Die für jede Emulsion optimale Zusatzmenge kann in einfacher Weise durch die üblichen Testversuche ermittelt werden.

Zusätzliche Stabilisierungsmittel können grundsätzlich den fotografischen Materialien bzw. Emulsionen vor, nach oder zu den Zeitpunkten zugesetzt werden, an denen auch die erfindungsgemäßen Stabilisierungsmittel zugesetzt werden.

Für die vorliegende Erfindung sind die üblichen Silberhalogenidemulsionen geeignet. Diese können als Silberhalogenid, Silberchlorid, Silberbromid oder Gemische davon, eventuell mit einem geringen Gehalt an Silberjodid bis zu 10 Mol-%, enthalten.

Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können auch chemisch sensibilisiert werden, z. B. durch Zusatz schwefelhaltiger Verbindungen bei der chemischen Reifung, beispielsweise Allylisothiocyanat, Allylthioharnstoff und Natriumthiosulfat. Als chemische Sensibilisatoren können ferner auch Reduktionsmittel, z. B. die in den belgischen Patentschriften 493 464 oder 568 687, beschriebenen Zinnverbindungen, ferner Polyamine wie Diethylentriamin oder Aminomethylsulfinsäurederivate, z. B. gemäß der belgischen Patentschrift 547 323, verwendet werden. Geeignet als chemische Sensibilisatoren sind auch Edelmetalle bzw. Edelmetallverbindungen wie Gold, Platin, Palladium, Iridium, Ruthenium oder Rhodium. Es ist ferner möglich, die Emulsionen mit Polyalkylenoxidderivaten zu sensibilisieren, z. B. mit Polyethylenoxid eines Molekulargewichts zwischen 1 000 und 20 000, ferner mit Kondensationsprodukten von Alkylenoxiden und Alkoholen, aliphatischen Carbonsäuren, aliphatischen Aminen, aliphatischen Diaminen und Amiden.

Die Emulsionen können auch optisch sensibilisiert sein, z. B. mit den üblichen Polymethinfarbstoffen, wie Neutrocyaninen, basischen oder sauren Carbocyaninen, Rhodacyaninen, Hemicyaninen, Styrylfarbstoffen, Oxonolen und ähnlichen. Derartige Sensibilisatoren sind in dem Werk von F. M. Hamer «The Cyanine Dyes and related Compounds», (1964), beschrieben.

Eine besonders vorteilhafte Wirkung zeigen die erfindungsgemäßen Stabilisatoren bei farbkupplerhaltigen Silberhalogenidemulsionen. Denn durch die Anwesenheit der Farbkuppler wird die Wirkung bekannter anderer Stabilisatoren oft sehr stark reduziert, so daß es beim Vorliegen von farbkupplerhaltigen Silberhalogenidemulsionen besonders schwierig ist, eine befriedigende Lagerstabilität und eine ausreichende Schleierfreiheit bei längeren Entwicklungszeiten oder höheren Entwicklungstemperaturen

zu erreichen. Dagegen haben die erfindungsgemäß zu verwendenden Stabilisatoren auch bei farbkupplerhaltigen Silberhalogenidemulsionen eine hervorragende stabilisierende Wirkung bei längerer Lagerung und bei einer Entwicklung bei höheren Temperaturen.

Das erfindungsgemäß hergestellte fotografische Material kann die üblichen Farbkuppler enthalten, die in der Regel den Silberhalogenidschichten selbst einverleibt sind. So enthält die rotempfindliche Schicht beispielsweise einen nicht-diffundierenden Farbkuppler zur Erzeugung des blaugrünen Teilfarbenbildes, in der Regel einen Kuppler vom Phenol- oder α-Naphtholtyp. Die grünempfindliche Schicht enthält mindestens einen nicht-diffundierenden Farbkuppler zur Erzeugung des purpurnen Teilfarbenbildes, wobei üblicherweise Farbkuppler vom Typ des 5-Pyrazolons oder des Indazolons Verwendung finden. Die blauempfindliche Schichteinheit schließlich enthält mindestens einen nicht-diffundierenden Farbkuppler zur Erzeugung des gelben Teilfarbenbildes, in der Regel einen Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichung « Farbkuppler » von W. Pelz in « Mitteilungen aus den Forschungslaboratorien der Agfa, Leverkusen/München », Band III (1961) und K. Venkataraman in « The Chemistry of Synthetic Dyes », Vol. 4, 341 bis 387, Academic Press, 1971, hingewiesen.

Als weitere nicht-diffundierende Farbkuppler können 2-Äquivalentkuppler verwendet werden ; diese enthalten in der Kupplungsstelle einen abspaltbaren Substituenten, so daß sie zur Farbbildung nur zwei Äquivalente Silberhalogenid benötigen im Unterschied zu den üblichen 4-Äquivalentkupplern. Zu den einsetzbaren 2-Äquivalentkupplern gehören beispielsweise die bekannten DIR-Kuppler, bei denen der abspaltbare Rest nach Reaktion mit Farbentwickleroxidationsprodukten als diffundierender Entwicklungsinhibitor in Freiheit gesetzt wird. Weiterhin können zur Verbesserung der Eigenschaften des fotografischen Materials die sogenannten Weißkuppler eingesetzt werden.

In besonders vorteilhafter Weise können die erfindungsgemäßen Stabilisatoren zusammen mit 2-Äquivalent-α-acylacetamid-Gelbkupplern verwendet werden. Besonders vorteilhafte Kuppler dieser Art sind Pivaloyl-Gelbkuppler der Formel (II)

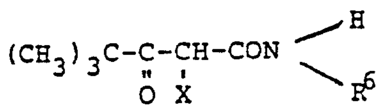

(II)

worin bedeuten :

$R^6$ eine Arylgruppe (insbesondere eine Phenyl-, Naphthylgruppe oder dgl.) oder eine heterocyclische Gruppe (insbesondere eine Thienyl-, Benzothienyl-, Furyl-, Pyranyl-, Pyrrolyl-, Imidazolyl-, Pyrazolyl-, Pyridyl-, Pyrimidyl-, Pyridazyl-, Indolyl-, Indazolyl-, Chinolyl-, Oxazolyl-, Pyrrolidyl-, Benzoimidazolyl-, Naphthoimidazolyl-, Benzoxazolyl-, Naphthoxazolyl-, Thiazolyl-, Benzothiazolyl-, Naphthothiazolyl-, Selenazolyl-, Benzoselenazolylgruppe oder dgl.) und

X einen Rest der Formel

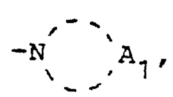

worin $A_1$ eine Gruppe von Nichtmetallatomen darstellt, die zur Bildung eines 4- bis 6-gliedrigen, Stickstoff enthaltenden heterocyclischen Ringes erforderlich sind, der außerdem ein Stickstoff-, Sauerstoff- oder Schwefebatom als ein Heteroatom sowie einen Substituenten aufweisen kann.

Die nicht-diffundierenden Farbkuppler und farbgebenden Verbindungen werden den lichtempfindlichen Silberhalogenidemulsionen oder sonstigen Gießlösungen nach üblichen bekannten Methoden zugesetzt. Wenn es sich um wasser- oder alkalilösliche Verbindungen handelt, können sie den Emulsionen in Form von wäßrigen Lösungen, gegebenenfalls unter Zusatz von mit Wasser mischbaren organischen Lösungsmitteln wie Ethanol, Aceton oder Dimethylformamid, zugesetzt werden. Soweit es sich bei den nicht-diffundierenden Farbkupplern und farbgebenden Verbindungen um wasser- bzw. alkaliunlösliche Verbindungen handelt, können sie in bekannter Weise emulgiert werden, z. B indem eine Lösung dieser Verbindungen in einem niedrigsiedenden organischen Lösungsmittel direkt mit der Silberhalogenidemulsion oder zunächst mit einer wäßrigen Gelatinelösung vermischt wird, worauf das organische Lösungsmittel in üblicher Weise entfernt wird. Ein so erhaltenes Gelatineemulgat der jeweiligen Verbindung wird anschließend mit der Silbernalogenidemulsion vermischt. Gegebenenfalls verwendet man zur Einemulgierung derartiger hydrophober Verbindungen zusätzlich noch sogenannte Kupplerlösungsmittel oder Ölformer ; das sind in der Regel höhersiedende organische Verbindungen, die die in den Silberhalogenidemulsionen zu emulgierenden nicht-diffundierenden Farbkuppler und Entwicklungsinhibitor abspaltenden Verbindungen in Form öliger Tröpfchen einschließen. Verwiesen sei in diesem Zusammenhang beispielsweise auf die US-Patentschriften 2 322 027 ; 2 533 514 ; 3 689 271 ; 3 764 336 und 3 765 897.

Die fotografischen Materialien können mit üblichen Schwarz-Weiß-Entwicklern, z. B. Hydrochinon, Brenzkatechin, p-Methylaminophenol und 1-Phenyl-3-pyrazolidon und mit Farbentwicklersubstanzen,

insbesondere vom Typ des p-Phenylendiamins, entwickelt werden, z. B. mit N,N-Dimethyl-p-phenylendiamin, 4-Amino-3-methyl-N-ethyl-N-methoxyethylanilin, 2-Amino-5-diethylaminotoluol, N-Butyl-N-ω-sulfobutyl-p-phenylendiamin, 2-Amino-5-(N-ethyl-N-β-methansulfonamidethyl-amino)-toluol, N-Ethyl-N-β-hydroxyethyl-p-phenylendiamin, N,N-Bis-(β-hydroxyethyl)-p-phenylendiamin, 2-Amino-5-(N-ethyl-N-β-hydroxyethylamino-)-toluol. Weitere brauchbare Farbentwickler sind beispielsweise beschrieben in J. Amer. Chem. Soc. *73*, 3100 (1951).

Die Schichten des fotografischen Materials können in der üblichen Weise gehärtet sein, beispielsweise mit Formaldehyd oder halogensubstituierten Aldehyden, die eine Carboxylgruppe enthalten, wie Mucobromsäure, Diketonen, Methansulfonsäureester, Dialdehyden und dergleichen. Weiterhin können die fotografischen Schichten mit Härtern des Epoxidtyps, des heterocyclischen Ethylenimins oder des Acryloyltyps gehärtet werden. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind. Es ist ferner möglich, die fotografischen Schichten bzw. die farbfotographischen Mehrschichtenmaterialien mit Härtern der Diazin-, Triazin- oder 1,2-Dihydrochinolin-Reihe zu härten. Beispiele derartiger Härter sind Alkyl- oder Arylsulfonylgruppen-haltige Diazinderivate, Derivate von hydrierten Diazinen oder Triazinen, wie z. B. 1,3,5-Hexahydrotriazin, Fluor-substituierte Diazinderivate, wie z. B. Fluorpyrimidin, Ester von 2-substituierten 1,2-Dihydrochinolin- oder 1,2-Dihydroisochinolin-N-carbonsäuren. Brauchbar sind weiterhin Vinylsulfonsäurehärter, Carbodiimid- oder Carbamoylhärter, wie z. B. in den deutschen Offenlegungsschriften 2 263 602, 2 225 230 und 1 808 685, der französischen Patentschrift 1 491 807, der deutschen Patentschrift 872 153 und der DDR-Patentschrift 7218, beschrieben. Weitere brauchbare Härter sind beispielsweise in der britischen Patentschrift 1 268 550 beschrieben.

## Beispiele

Die folgenden Beispiele erläutern die Erfindung, ohne daß diese auf die Ausführungsformen in den Beispielen beschränkt ist.

In diesen Beispielen werden als Vergleichssubstanzen die folgenden bekannten Stabilisatoren verwendet :

### Übersicht

| | Verbindung | bekannt aus |
|---|---|---|
| A | 3-Acetylthio-5-amino-1,2,4-triazol | |
| B | 3-Acetylthio-4-acetyl-5-amino-1,2,4-triazol | |
| C | 3-Ethoxycarbonylthio-5-ethoxycarbonylamino-1,2,4-triazol | DE-C-1 522 363 Verbindung 27 |
| D | 1-Phenyl-5-mercapto-1,2,3,4-tetrazol | |
| E | 3-Mercapto-4-amino-1,2,4-triazol | JP-A-78/17492 und |
| F | 3-Mercapto-4-acetylamino-1,2,4-triazol | JP-A-75/539 |
| G | 3-Mercapto-4-amino-5-methyl-1,2,4-triazol | JP-A-75/539 |
| H | 3-Mercapto-4-acetylamino-5-methyl-1,2,4-triazol | JP-A-75/539 |
| I | 3-Mercapto-1-phenyl-5-acetylamino-1,2,4-triazol | DE-A-2 308 530 |
| J | 4-(p-Sulfo-anilino)-5-methyl-3-mercapto-1,2,4-triazol | DE-C-1 183 371 |
| K | 1-Phenyl-3-mercapto-1,2,4-triazol | |

## Beispiel 1

Eine hochempfindliche Bromjodsilberemulsion mit 5 Mol-% Jodid und einem Gelatine-Silber-Verhältnis von 1,2 und einem Gehalt von 85 g Silbernitrat pro kg Emulsion wurde mit Schwefel- und Goldverbindungen zur optimalen Empfindlichkeit gereift.

Der Emulsionsansatz wurde in mehrere Teile geteilt und pro kg Emulsion wurden folgende Substanzen zugesetzt :

| | |
|---|---|
| 4-Hydroxy-6-methyl-1,3,3a,7-tetraazainden<br>1 %ig, wäßrig-alkalische Lösung | 150 mg |
| Saponin<br>10 %ig, gelöst in Wasser | 3,5 g |

und die aus der folgenden Tabelle ersichtlichen erfindungsgemäßen Substanzen (1 %ig gelöst in Aceton oder Methanol) in den angegebenen Mengen. Die Mengen wurden so bemessen, daß noch keine stärkere Beeinträchtigung der Empfindlichkeit auftrat.

Die Emulsionen wurden anschließend auf einen Celluloseacetatträger vergossen und getrocknet (Auftag 6,8 bis 7.0 g, berechnet als Silbernitrat pro $m^2$). Auf die Emulsionsschicht wurde jeweils eine Schutzschicht, die ein geeignetes Härtungsmittel und ein Netzmittel enthielt, mit einer Auftragsdicke von 2 g Gelatine/$m^2$ aufgetragen. Die Proben wurden einer Frischprüfung und einer Heizschrankprüfung nach einer Lagerung von 3 Tagen bei 60 °C unterzogen.

Die Proben wurden dann in einem Sensitometer hinter einem Graukeil belichtet und bei 38 °C 6 Minuten lang in einem Entwickler I der folgenden Zusammensetzung entwickelt. Dieser einen Silberkomplexbildner enthaltenden Entwickler ist typisch für einen Schwarz-Weiß-Entwickler für die Umkehrverarbeitung von fotografischen Materialien.

Entwickler I

| | |
|---|---|
| 4-Hydroxymethyl-4-methyl-1-phenyl-3-pyrazolidon | 1,4 g |
| Natriumhydrogencarbonat sicc. | 12 g |
| Diethylenglykol | 12 ml |
| Kaliumhydroxid 45 % | 7 ml |
| Kaliumjodid | 4,5 mg |
| Hydrochinonsulfonsaures-Kalium | 22 g |
| Kaliumcarbonat sicc. | 14 g |
| Kaliumsulfit 45 % | 44 ml |
| Kaliumrhodanid sicc. | 1 g |
| Kaliumbromid | 2,2 g |
| Nitrilo-trimethylentriphosphonsaures-$Na_6$-Salz | 1 ml |

mit Wasser auf 1 l auffüllen und auf pH 9,6 einstellen.

Anschließend folgt ein Stoppbad bestehend aus 10 g Natriumacetat sicc. und 20 g 96 %igem Eisessig in 1 l Wasser. Anschließend wird mit 15 %iger Ammoniumthiosulfat- und 1 %iger Natriumsulfitlösung fixiert und danach gewässert. Die Ergebnisse der sensitometrischen Auswertung sind aus Tabelle 3 ersichtlich.

Zur Schleierprüfung wird eine unbelichtete Probe 6 Minuten lang in dem Entwickler I bei 38 °C entwickelt.

Die weitere Verarbeitung erfolgt wie oben angegeben. Als Maß für die erhaltene Verschleierung wird die « prozentuale Verschleierung » in Tabelle 3 angegeben ; die prozentuale Verschleierung ergibt sich aus dem Verhältnis von entwickeltem Silber (als Silbernitrat) dividiert durch Silber (als Silbernitrat) vor der Verarbeitung und Multiplikation dieses Verhältnisses mit 100.

Aus der Tabelle ist ersichtlich, daß die Substanzen den Schleier, insbesondere den Heizschrankschleier, vermindern und daher als Antischleiermittel für Umkehrmaterialien geeignet sind.

Tabelle 3

| Verbindung-Nr. | mg/kg | Frischprüfung Empfindlich-keit *) | γ | % Verschleie-rung | Heizschrankprüfung Empfindlichkiet *) | γ | % Verschleie-rung |
|---|---|---|---|---|---|---|---|
| Kontroll-probe | — | 41,0 | 0,73 | 41 | 40,6 | 0,68 | 57 |
| 1.1 | 40 | + 0,4° | 0,69 | 18 | + 1,4° | 0,68 | 26 |
| 1.8 | 21 | ± 0° | 0,69 | 20 | + 0,8° | 0,67 | 28 |
| 1.8 | 43 | + 0,7° | 0,64 | 11 | + 1,3° | 0,65 | 13 |
| 1.9 | 63 | + 0,7° | 0,75 | 26 | + 0,4° | 0,69 | 25 |
| 1.9 | 127 | − 0,8° | 0,71 | 13 | ± 0° | 0,67 | 12 |
| 1.10 | 75 | ± 0° | 0,75 | 15 | + 1,0° | 0,73 | 17 |

*) 3° = 1 Blende.

Beispiel 2

Zu 1 kg einer grünsensibilisierten Bromjodsilber-Emulsion mit einem Silber (berechnet als Silbernitrat)/Gelatineverhältnis von 1 : 0,4 und einem Gehalt von 0,91 Mol Silberhalogenid pro kg Emulsion mit 5 Mol-% Jodid wurden 1,2 g 4-Hydroxy-6-methyl-1,3,3a,7-tetraazainden in wäßrig-alkalischer Lösung zugesetzt und die so erhaltene Emulsion in mehrere gleiche Teile geteilt und den einzelnen

Proben die aus der folgenden Tabelle 4 ersichtlichten erfindungsgemäßen Verbindungen in Aceton oder Alkohol gelöst in den angegebenen Mengen zugesetzt. Vor dem Beguß wurden der Emulsion pro kg zugesetzt :

75 g einer 5 %igen Gelatinelösung ;
109 g einer 11,1 %igen Kupplerdispersion des folgenden Purpurkupplers

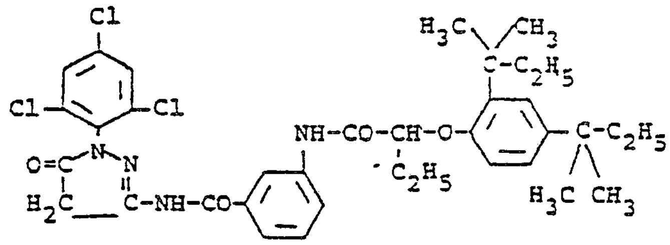

sowie Netzmittel in wäßriger Lösung und 1 180 ml Wasser.

Die Emulsionen wurden auf die aus einer Silberdispersion bestehende Lichthofschutzschicht eines Celluloseacetatträgers mit einem Silberauftrag entsprechend 5,0 bis 5,2 g $AgNO_3/m^2$ vergossen.

Auf die Emulsionsschicht wurde jeweils eine Schutzschicht, die ein geeignetes Härtungsmittel und ein Netzmittel enthielt, mit einer Auftragsdicke von 2 g Gelatine/$m^2$ aufgetragen.

Die Proben wurden einer Frisch- und einer Heizschrankprüfung von 3 Tagen bei 38 °C und 60 % rel. Luftfeuchtigkeit unterzogen.

Die Proben wurden dann in einem Sensitometer hinter einem Stufenkeil belichtet und in dem folgenden Entwickler II bei 38 °C 3 1/4 Minuten entwickelt.

Entwickler II

| | |
|---|---|
| 1-Hydroxyethan-1,1-diphosphonsäure-$Na_2$-salz | 2 g |
| Ethylendiamin-N,N,N',N'-tetraessigsäure | 2 g |
| Kaliumcarbonat sicc. | 34,1 g |
| Natriumhydrogencarbonat sicc. | 1,55 g |
| Natriumdisulfit sicc. | 0,28 g |
| Natriumsulfit sicc. | 3,46 g |
| Kaliumbromid | 1,34 g |
| Hydroxylaminsulfat | 2,4 g |
| 4-Amino-3-methyl-N-ethyl-N-(β-hydroxyethyl)-anilin | 4,7 g |

mit Wasser auf 1 l auffüllen.

Die weitere Verarbeitung umfaßt die folgenden Bäder :

| | |
|---|---|
| Stoppbad | 1 Minute bei 38 °C ; |
| Bleichbad | 3 1/4 Minuten bei 38 °C ; |
| Wässern | 3 1/2 Minuten bei 38 °C ; |
| Fixierbad | 3 1/4 Minuten bei 38 °C ; |
| Wässern | 5 Minuten bei 38 °C. |

Die verwendeten Stopp-, Bleich- und Fixierbäder entsprechen den üblicherweise verwendeten (British Journal of Photography, *1974,* Seiten 597 und 598).

Die erhaltenen Ergebnisse sind aus Tabelle 4 ersichtlich.

Die Substanzen vermindern den sehr hohen Schleier um mehr als 50 % ohne die Empfindlichkeit und die Gradation zu vermindern und verbessern die Lagerstabilität des fotografischen Materials.

(Siehe Tabelle 4, Seite 12 f.)

Tabelle 4

| Verbindung-Nr. | mg/kg | Frischprüfung Empfindlichkeit*) | γ | Schleier | Heizschrankprüfung Empfindlichkeit*) | γ | Schleier |
|---|---|---|---|---|---|---|---|
| Kontrollprobe | — | 39,1 | 0,75 | 1,16 | 38,5 | 0,68 | 1,17 |
| 1.1 | 40 | − 0,7° | 1,07 | 0,36 | − 0,4° | 0,99 | 0,44 |
| 1.9 | 127 | ± 0° | 1,03 | 0,39 | − 0,1° | 1,01 | 0,47 |
| 1.10 | 149 | − 0,3° | 1,09 | 0,27 | − 0,1° | 1,11 | 0,31 |

*) 3° = 1 Blende.

Beispiel 3

125 g einer Silberbromidemulsion mit hoher Blauempfindlichkeit und einem Silbernitratgehalt und Gelatinegehalt von 100 g/1 000 g Emulsion wurde in Gegenwart von 7 mg 4-Hydroxy-6-methyl-1,3,3a,7-tetraazatriazainden in wäßrig-alkalischer Lösung gelöst aufgeschmolzen und dann ein geeigneter Blausensibilisator zugesetzt. Danach wurden die erfindungsgemäßen Substanzen und die Vergleichssubstanzen in Methanol oder Aceton gelöst in den in der Tabelle 5 angegebenen Mengen zugegeben. Anschließend wurde der folgende 2-Äquivalentgelbkuppler

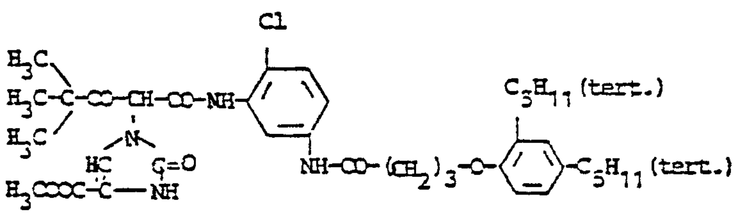

in Trikresylphosphat und Ethylacetat gelöst und unter Verwendung von Di-sek. butylnaphthalinsulfonat in einer Gelatinelösung emulgiert und dann das Ethylacetat abdestilliert. Von diesem Emulgat, das ca. 5 % Kuppler enthielt, wurde dann soviel zugesetzt, daß 18,7 g Kuppler in dem Ansatz enthalten war. Weiterhin wurde dem Ansatz noch 0,8 g Dioctylhydrochinon in Form eines Trikresylphosphatemulgats und ein Netzmittel zugesetzt.

Jeder dieser Ansätze wird auf ein substriertes polyethylenbeschichtetes Papier mit einem Auftrag von 0,55 g Silber berechnet als $AgNO_3/m^2$, aufgebracht. Auf die Emulsionsschicht wurde eine Schutzschicht mit 6,4 g $Gelatine/m^2$ aufgetragen.

Das Schichtpaket wurde dann mit einem geeigneten Härtungsmittel überschichtet.

Die so hergestellten Schichten wurden hinter einem Keil der Abstufung $\sqrt[3]{2}$ und einem Blaufilter belichtet und dann in folgender Weise entwickelt :

| | | |
|---|---|---|
| Farbentwickler III | 33 °C | 3,5 Minuten |
| Bleichfixierbad | 33 °C | 1,5 Minuten |
| Wässerung | 33 °C | 3 Minuten |

Die Verarbeitungsbäder wurden nach den folgenden Ansatzrezepten hergestellt :

Entwickler III

| | | |
|---|---|---|
| 900 | ml | Wasser |
| 15 | ml | Benzylalkohol |
| 15 | ml | Ethylenglykol |
| 3 | g | Hydroxylaminsulfat |
| 4,5 | g | 3-Methyl-4-amino-N-ethyl-N-(β-methansulfonamidoethyl)-anilinsulfat |
| 32 | g | Kaliumcarbonat sicc. |
| 2 | g | Kaliumsulfit sicc. |
| 0,6 | g | Kaliumbromid |
| 1 | g | Dinatriumsalz der 1-Hydroxyethylidin-1,1-diphosphonsäure |

mit Wasser auf 1 l auffüllen und auf pH 10,2 einstellen.

Bleichfixierbad

700 ml Wasser
35 ml Ammoniaklösung (28 %ig)
30 g Ethylendiamin-N,N,N',N'-tetraessigsäure
15 g Natriumsulfit sicc.
100 g Ammoniumthiosulfat sicc.
60 g Natrium-(ethylendiamintetraacetat)-eisen-(III)-komplex

mit Wasser auf 1 l auffüllen und auf pH 7 einstellen.

Nach der Entwicklung wurden die gelben Farbkeile ausgemessen. Bei der Auswertung ergaben sich dann die Zahlenwerte der folgenden Tabelle 5 :

Tabelle 5

| Verbindung | Konzentr. mg/100 g $Ag/NO_3$ | $D_{min}$ | $D_{max}$ | Empfindlichkeit *) D = 0,3 | D = 1,0 |
|---|---|---|---|---|---|
| Kontrollprobe | — | 0,22 | 1,67 | 6,5 | 12,0 |
| 1.1 | 20 | 0,11 | 1,63 | 9,3 | 13,0 |
| 1.2 | 100 | 0,11 | 1,62 | 9,1 | 13,5 |
| 1.4 | 50 | 0,11 | 1,62 | 8,5 | 12,3 |
| 1.8 | 40 | 0,11 | 1,62 | 8,9 | 13,1 |
| 1.9 | 100 | 0,11 | 1,65 | 8,9 | 13,1 |
| 1.10 | 100 | 0,11 | 1,72 | 8,4 | 12,5 |
| 1.11 | 100 | 0,11 | 1,69 | 9,0 | 13,0 |
| 1.13 | 50 | 0,10 | 1,59 | 8,7 | 12,3 |
| 1.14 | 150 | 0,11 | 1,67 | 8,6 | 12,1 |
| 1.24 | 100 | 0,10 | 1,73 | 8,5 | 12,3 |
| A | 50 | 0,16 | 1,40 | 14,0 | 18,5 |
| B | 100 | 0,11 | 1,53 | 13,5 | 17,4 |
| C | 200 | 0,11 | 1,70 | 11,0 | 14,9 |
| D | 60 | 0,11 | 1,65 | 8,2 | 12,5 |
| E | 100 | 0,16 | 1,55 | 14,0 | 18,0 |
| F | 50 | 0,10 | 1,56 | 12,9 | 16,1 |
| H | 30 | 0,12 | 1,56 | 9,9 | 14,0 |

*) Empfindlichkeit = xte Stufe bei Dichte 0,3 bzw. 1,0.
Je höher der Wert, desto niedriger ist die Empfindlichkeit.

Eine Vergrößerung des angegebenen Wertes um den Faktor $\sqrt[3]{2}$ entspricht einer Verringerung der Empfindlichkeit um 1 DIN.

Man kann sehr deutlich erkennen, daß die Stabilisatoren der ersten Gruppe (1.1-1.24) bei einem guten Schleierwert $D_{min}$ zwar eine leicht gedrückte, aber noch befriedigende Empfindlichkeit haben, während die Verbindungen der zweiten Gruppe (A/B/C/E/F/H) bei guten bzw. noch nicht ausreichend verbesserten Schleierwerten sehr starke Empfindlichkeitsrückgänge zeigen und deshalb nicht brauchbar sind. Außerdem behindern wie im folgenden Beispiel 4 gezeigt wird die Verbindungen D, I, J, K die Bleichung.

## Beispiel 4

Dieses Beispiel zeigt einen Vergleich bekannter mercaptogruppenhaltiger Stabilisatoren mit den erfindungsgemäßen bei der Bleichung.

Hierzu wird eine Silberfiltergelb-Gelatinelösung mit den in der Tabelle 6 angegebenen Mengen eines Stabilisators (Mol Stabilisator pro Mol Silber) versetzt, und nach Zufügen von Saponin als Netzmittel zu Schichten mit einer Dichte von 1.6-2.1 auf eine substrierte polyethylen beschichtete Papierunterlage vergossen.

Die Proben werden 5 Minuten lang in einer Pufferlösung bei pH 10 behandelt, 5 Minuten gewässert und dann in einem Bleichfixierbad der folgenden Zusammensetzung 1 Minute bei 20 °C gebadet :

20 g Na-Salz des Fe III-Komplexes der Ethylendiamintetraessigsäure

10 g Natriumsulfit sicc.
5 g Kaliumdihydrogenphosphat sicc.
100 g Ammoniumthiosulfat sicc.

mit Wasser auf 1 l auffüllen, pH 6,0.

Danach wurden die Keile eine Minute in fließendem Wasser gespült und getrocknet. Vor und nach der Behandlung wurde die Dichte der Keile gemessen, die Werte sind in der Tabelle 6 zusammengefaßt.

Tabelle 6

| Verbindung-Nr. | Konzentration | Unbehandelt | Gebleicht |
|---|---|---|---|
| 1.1 | $8 \cdot 10^{-2}$ | 2,07 | 0,07 |
| 1.2 | $3,8 \cdot 10^{-2}$ | 1,82 | 0,11 |
| 1.3 | $8 \cdot 10^{-2}$ | 1,97 | 0,21 |
| 1.6 | $7,2 \cdot 10^{-2}$ | 1,87 | 0,12 |
| 1.8 | $7,5 \cdot 10^{-2}$ | 1,73 | 0,06 |
| 1.11 | $7,1 \cdot 10^{-2}$ | 1,78 | 0,05 |
| 1.15 | $3,6 \cdot 10^{-2}$ | 1,44 | 0,06 |
| 1.22 | $3,8 \cdot 10^{-2}$ | 1,59 | 0,02 |
| 1.24 | $2,8 \cdot 10^{-2}$ | 1,66 | 0,23 |
| D | $7 \cdot 10^{-2}$ | 1,93 | 2,06 |
| E | $7 \cdot 10^{-2}$ | 2,14 | 0,04 |
| H | $7 \cdot 10^{-2}$ | 1,79 | 0,09 |
| I | $7 \cdot 10^{-2}$ | 1,88 | 1,78 |
| J | $7 \cdot 10^{-2}$ | 1,91 | 2,38 |
| K | $6,8 \cdot 10^{-2}$ | 1,97 | 2,06 |
| Kontrollprobe | — | 1,64 | 0,19 |

Die Vergleichsverbindungen D, I, J, K hemmen die Bleichung. Die als Bleichbeschleuniger bekannten Vergleichsverbindungen H, E wirken ebenso wie die erfindungsgemäßen Verbindungen 1.1, 1.2, 1.6, 1.8, 1.11, 1.15, 1.22 bleichbeschleunigend, drücken aber wie im Beispiel 3 gezeigt die Empfindlichkeit und/oder zeigen nur eine geringe schleiervermindernde Wirkung. Die Verbindungen 1.3 und 1.24 wirken nicht bzw. kaum bleichhemmend.

Beispiel 5

Eine in der üblichen Weise hergestellte Silber-Chlor-Brom-Jodemulsion mit 2 % AgCl und 0,5 % AgJ, die eine enge Kornverteilung aufweist, wird nach dem Flocken und Waschen in einer Gelatinelösung, derart aufgenommen, daß das Mengenverhältnis Silber zu Gelatine 1 : 1 ist und pro kg Emulsion etwa 100 g Silberhalogenid enthält. Der pH-Wert wird zum Reifen auf 6,5 und der pAg auf 8,9 eingestellt. Nach dem Aufschmelzen der Emulsion bei 40 °C werden als Reifzusätze Goldsalze und Thiosulfat zugegeben. Vor dem Hochheizen auf die Reiftemperatur wird die Emulsion in mehrere Teile aufgeteilt. Während die erste Probe ohne weitere Zusätze gereift wird, werden die erfindungsgemäßen Substanzen als Reifstabilisatoren in Alkoholen oder Aceton gelöst zugegeben und zwar in den in der Tabelle 7 angegebenen Mengen. Die Emulsionen werden ausgereift (ca. 100 min bei 48 °C). Anschließend werden die Emulsionen mit den im Beispiel 3 beschriebenen Zusätzen versetzt. Die Hälfte der gießfertigen Emulsion wird 24 Stunden bei 40 °C digeriert. Es werden vor dem Beguß lediglich noch 15 ml einer 3 %igen Lösung von Triacrylformal und ein Netzmittel zugesetzt. Die erhaltene Emulsion wird auf einen Träger aufgetragen. Als Schutzschicht wurde eine Gelatineschutzschicht mit 1,8 g Gelatine pro $m^2$ aufgebracht, die ebenfalls mit 15 ml einer 3 %igen Triacrylformallösung auf 45 g Gelatine gehärtet wurde. Anschließend wurden die Proben 2 Tage bei 60 °C und 60 % relativer Feuchtigkeit gelagert.

Die Verarbeitung erfolgte nach der Heizschranklagerung der Proben in den im Beispiel 3 angegebenen Bädern.

Die Verarbeitungsbedingungen wurden dabei in folgender Weise geändert.

| | | |
|---|---|---|
| Entwickler | 25 °C | 7 Minuten |
| Stoppbad wie in Beispiel 1 | 20 °C | 1 Minute |
| Wässerung | kalt | 1 Minute |
| Bleichfixierbad | 20 °C | 2 Minuten |
| Wässerung | kalt | 4 Minuten |

Außerdem wurden die Langzeitschleier bei folgenden Verarbeitungsbedingungen bestimmt.

| | | |
|---|---|---|
| Entwickler | 25 °C | 5/10/15 Minuten |
| Stoppbad | 20 °C | 1 Minute |
| Wässerung | kalt | 1 Minute |
| Bleichfixierbad | 20 °C | 3 Minuten |
| Wässerung | kalt | 4 Minuten |

Die Belichtung der Keile erfolgte hinter einem $\sqrt[3]{2}$ Keil und einem Blaufilter. Die Ergebnisse sind aus der folgenden Tabelle 7 zu ersehen.

Tabelle 7

| Verbindung-Nr. und Menge pro 100 g $AgNO_3$ | 7 min Entwicklungszeit E | $\gamma$ | $D_{max}$ | Langzeitschleier bei einer Entwicklungszeit von 5′ | 10′ | 15′ |
|---|---|---|---|---|---|---|
| Ohne | 100 | 1,60 | 1,88 | 0,45 | 0,49 | 0,62 |
| | 100 | 1,60 | 1,88 | 0,32 | 0,46 | 0,56*) |
| 17 mg Nr. 1.2 | 75 | 1,75 | 1,90 | 0,14 | 0,18 | 0,28 |
| | 60 | 2,00 | 1,69 | 0,11 | 0,15 | 0,21*) |
| 25 mg Nr. 1.2 | 70 | 1,60 | 1,82 | 0,17 | 0,15 | 0,21 |
| | 50 | 1,70 | 1,79 | 0,08 | 0,11 | 0,16*) |
| 17 mg Nr. 1.4 | 85 | 1,80 | 1,78 | 0,14 | 0,22 | 0,32 |
| | 75 | 1,70 | 1,68 | 0,07 | 0,11 | 0,21*) |
| 25 mg Nr. 1.4 | 80 | 1,70 | 1,85 | 0,13 | 0,17 | 0,23 |
| | 70 | 1,80 | 1,85 | 0,07 | 0,11 | 0,15*) |
| 17 mg Nr. 1.8 | 80 | 1,70 | 1,89 | 0,13 | 0,18 | 0,24 |
| | 70 | 1,85 | 1,64 | 0,11 | 0,18 | 0,27*) |
| 8 mg Nr. 1.10 | 90 | 1,80 | 1,78 | 0,14 | 0,22 | 0,32 |
| | 80 | 1,70 | 1,68 | 0,07 | 0,21 | 0,21*) |
| 12 mg Nr. 1.5 | 85 | 1,75 | 1,85 | 0,25 | 0,27 | 0,30 |
| | 80 | 1,75 | 1,80 | 0,23 | 0,24 | 0,26*) |
| 15 mg Nr. 1.22 | 75 | 1,65 | 1,70 | 0,15 | 0,19 | 0,23 |
| | 75 | 1,75 | 1,75 | 0,14 | 0,19 | 0,21*) |

Die mit einem *) gekennzeichneten Ergebnisse werden nach 24 Stunden Digestion erhalten.
E : Empfindlichkeit bei der Dichte D = 1,0.

**Ansprüche**

1. Lichtempfindliches fotografisches Material mit wenigstens einer lichtempfindlichen Silberhalogenidemulsionsschicht und gegebenenfalls weiteren Schichten, dadurch gekennzeichnet, daß es in wenigstens einer Schicht eine Verbindung der Formel (I) enthält

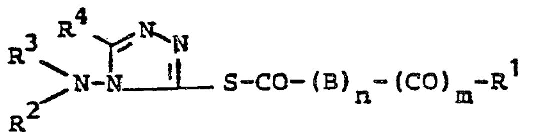

(I)

worin bedeuten

$R^1$ einen Alkyl-, Cycloalkyl-, Aryl- oder Aralkylrest oder einen substituierten über ein —S— Atom gebundenen Triazolrest,

$R^2$ H, Acyl, Alkyl oder —$COOR^5$,

$R^3$ H, Acyl oder —$COOR^5$,

$R^4$ H oder Alkyl,

$R^5$ Alkyl, Cycloalkyl, Aryl, Aralkyl,

n, m gleich oder verschieden, 0 oder 1,

B —O—, Alkylen, Arylen, Cycloalkylen oder Aralkylen, wobei diese organischen Reste an ein oder

zwei Stellen ein Sauerstoffatom tragen können
und wobei die angegebenen Substituenten ihrerseits mit Substituenten substituiert sein können, die für Stabilisatoren auf dem fotografischen Gebiet üblich sind.

2. Material nach Anspruch 1, dadurch gekennzeichnet, daß bedeuten :

$R^1$ einen Alkylrest mit 1 bis 6 C-Atomen, einen Cycloalkylrest mit 5 oder 6 C-Atomen, einen Phenylrest oder einen Benzylrest oder einen Rest der Formel

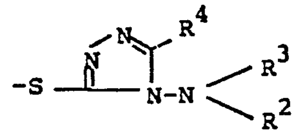

$R^2$ H, Acetyl, Alkyl mit 1 bis 4 C-Atomen oder $COOR^5$,
$R^3$ H oder Acyl,
$R^4$ H oder Alkyl mit 1 bis 4 C-Atomen,
$R^5$ Alkyl mit 1 bis 6 C-Atomen oder Cycloalkyl mit 5 oder 6 C-Atomen, oder Phenyl oder Benzyl.

3. Material nach Anspruch 1, dadurch gekennzeichnet, daß bedeuten :
$R^1$ Methyl, Ethyl, Isopropyl, Butyl oder einen Cyclohexylrest
$R^2$ H oder $COOR^5$,
$R^3$ Acetyl oder $COOR^5$
$R^4$ H, Methyl oder Ethyl,
$R^5$ Ethyl, Butyl oder Cyclohexyl.

4. Material nach Anspruch 1, dadurch gekennzeichnet, daß B bedeutet :

$-O-$
$-O-[CH_2]_n-O-$ mit $n = 1\text{-}4$
$-[CH_2]_{7n}-$ mit $n = 1\text{-}4$

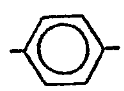 oder 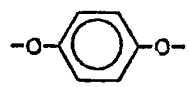

5. Material nach Anspruch 1, dadurch gekennzeichnet, daß die Verbindung der Formel (I) in einer Menge von 5 mg bis 500 mg pro Mol Silberhalogenid enthalten ist.

6. Material nach Anspruch 1, dadurch gekennzeichnet, daß wenigstens eine der folgenden Verbindungen enthalten ist :

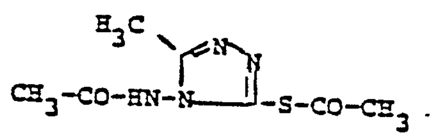

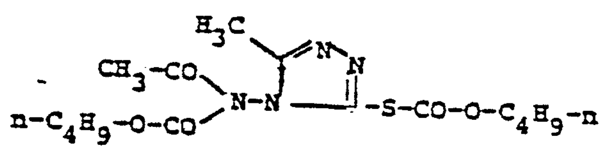

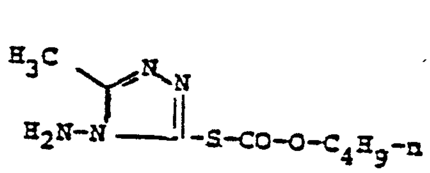

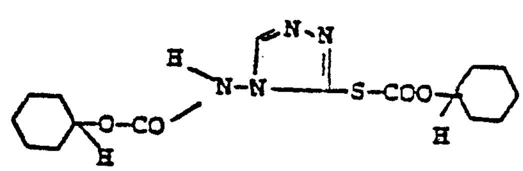

7. Verfahren zur Herstellung lichtempfindlicher silberhalogenidhaltiger fotografischer Materialien durch Fällen des Silberhalogenids, Reifung und Auftragen auf einen Schichtträger, dadurch gekennzeichnet, daß der Silberhalogenidemulsion vor dem Auftragen eine Verbindung der Formel (I) des Anspruchs 1 zugesetzt wird.

8. Verfahren nach Anspruch 7, dadurch gekennzeichnet, daß der Zusatz vor der chemischen Reifung erfolgt.

9. Verfahren zur Herstellung fotografischer Bilder durch bildmäßige Belichtung und Entwicklung eines fotografischen Aufzeichnungsmaterials, dadurch gekennzeichnet, daß ein Material nach Anspruch 1 verwendet wird.

10. Verbindungen der Formel (I)

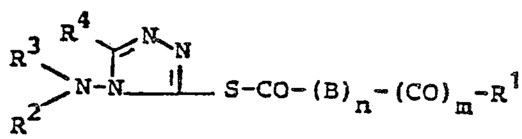

(I)

worin bedeuten

$R^1$ einen Alkyl-, Cycloalkyl-. Aryl- oder Aralkylrest oder einen substituierten über ein —S— Atom gebundenen Triazolrest,

$R^2$ H, Acyl, Alkyl oder —$COOR^5$,

$R^3$ H, Acyl oder —$COOR^5$,

$R^4$ H oder Alkyl,

$R^5$ Alkyl, Cycloalkyl, Aryl, Aralkyl,

n, m gleich oder verschieden, 0 oder 1,

B —O—, Alkylen, Arylen, Cycloalkylen oder Aralkylen, wobei diese organischen Reste an ein oder zwei Stellen ein Sauerstoffatom tragen können

und wobei die angegebenen Substituenten ihrerseits mit Substituenten substituiert sein können, die für Stabilisatoren auf dem fotografischen Gebiet üblich sind.

**Claims**

1. Light-sensitive photographic material containing at least one light-sensitive silver halide emulsion layer and optionally other layers, characterised in that it contains, in at least one layer, a compound of the formula (I)

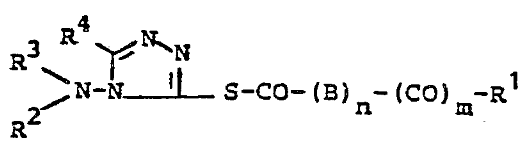

(I)

wherein

$R^1$ denotes an alkyl, cycloalkyl, aryl or aralkyl radical or a substituted triazole radical linked via an —S— atom,

$R^2$ denotes H, acyl, alkyl or —$COOR^5$,

$R^3$ denotes H, acyl or —$COOR^5$,

$R^4$ denotes H or alkyl,

$R^5$ denotes alkyl, cycloalkyl, aryl or aralkyl,

n and m, which are the same or different, denote 0 or 1, and

B denotes —O—, alkylene, arylene, cycloalkylene or aralkylene, it being possible for these organic radicals to carry an oxygen atom at one or two positions,

and it being possible for the indicated substituents in their turn to be substituted by substituents which are customary for stabilisers in the photographic field.

2. Material according to Claim 1, characterised in that

$R^1$ denotes an alkyl radical with 1 to 6 C atoms, a cycloalkyl radical with 5 or 6 C atoms, a phenyl radical or a benzyl radical or a radical of the formula

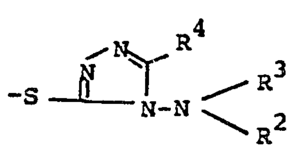

$R^2$ denotes H, acetyl, alkyl with 1 to 4 C atoms or $COOR^5$,
$R^3$ denotes H or acyl,
$R^4$ denotes H or alkyl with 1 to 4 C atoms,
and
$R^5$ denotes alkyl with 1 to 6 C atoms or cycloalkyl with 5 or 6 C atoms, or phenyl or benzyl,

3. Material according to Claim 1, characterised in that
$R^1$ denotes methyl, ethyl, isopropyl, butyl or a cyclohexyl radical,
$R^2$ denotes H or $COOR^5$,
$R^3$ denotes acetyl or $COOR^5$,
$R^4$ denotes H, methyl or ethyl, and
$R^5$ denotes ethyl, butyl or cyclohexyl,

4. Material according to Claim 1, characterised in that B denotes :

$—O—$
$—O—[CH_2]_n—O—$ in which $n = 1\text{-}4$
$—[CH_2]_n—$ in which $n = 1\text{-}4$

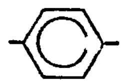

or

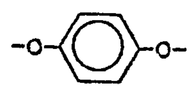

5. Material according to Claim 1, characterised in that it contains the compound of the formula (I) in a quantity of 5 mg to 500 mg per mol of silver halide.

6. Material according to Claim 1, characterised in that it contains at least one of the following compounds :

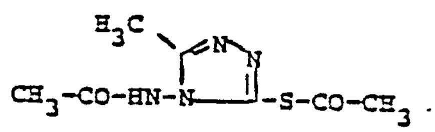

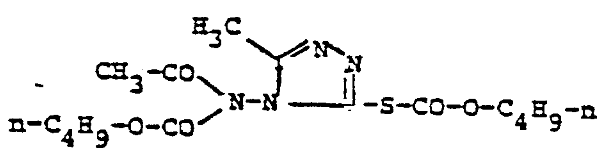

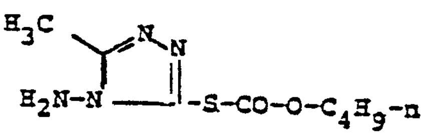

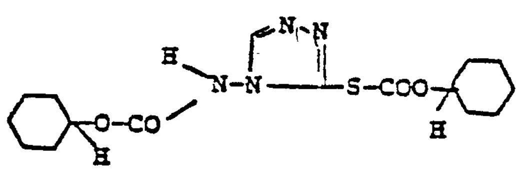

7. Process for the preparation of light-sensitive photographic materials containing silver halide by precipitation of the silver halide, ripening and application to a layer support, characterised in that a compound of the formula (I) of Claim 1 is added to the silver halide emulsion before the emulsion is applied.

8. Process according to Claim 7, characterised in that the addition is carried out before chemical ripening.

9. Process for the production of photographic images by imagewise exposure and development of a photographic recording material, characterised in that a material according to Claim 1 is used.

10. Compounds of the formula (I)

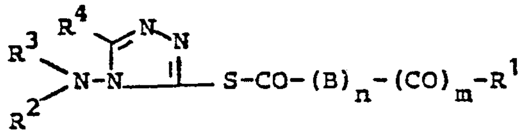

(I)

wherein

$R^1$ denotes an alkyl, cycloalkyl, aryl or aralkyl radical or a substituted triazole radical linked via an —S— atom,

$R^2$ denotes H, acyl, alkyl or —$COOR^5$,

$R^3$ denotes H, acyl or —$COOR^5$,

$R^4$ denotes H or alkyl,

$R^5$ denotes alkyl, cycloalkyl, aryl or aralkyl,

n and m, which are the same or different, denote 0 or 1, and

B denotes —O—, alkylene, arylene, cycloalkylene or aralkylene, it being possible for these organic radicals to carry an oxygen atom at one or two positions,

and it being possible for the indicated substituents in their turn to be substituted by substituents which are customary for stabilisers in the photographic field.

**Revendications**

1. Matériel photographique sensible à la lumière portant au moins une couche photosensible d'émulsion à l'halogénure d'argent et le cas échéant d'autres couches, caractérisé en ce qu'il comprend dans au moins une couche un composé de formule (I)

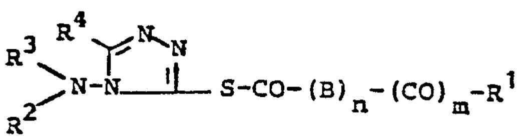

(I)

dans laquelle

$R^1$ désigne un reste alkyle, cycloalkyle, aryle ou aralkyle ou un reste triazole substitué en liaison par l'intermédiaire d'un atome —S—,

$R^2$ représente H, ou un reste acyle, alkyle ou —$COOR^5$,

$R^3$ représente H, un reste acyle, ou un reste —$COOR^5$,

$R^4$ représente H ou un reste alkyle,

$R^5$ est un reste alkyle, cycloalkyle, aryle, aralkyle,

n, m égaux ou différents, ont la valeur 0 ou 1

B représente —O—, un reste alkylène, arylène, cycloalkylène, ou aralkylène, ces restes organiques pouvant porter un atome d'oxygène en un ou deux sites,

et les substituants indiqués peuvent, quant à eux, être substitués avec des substituants qui sont classiques pour des stabilisants dans le domaine de la photographie.

2. Matériel suivant la revendication 1, caractérisé en ce que

$R^1$ est un reste alkyle ayant 1 à 6 atomes de carbone, un reste cycloalkyle ayant 5 ou 6 atomes de carbone, un reste phényle ou un reste benzyle ou un reste de formule

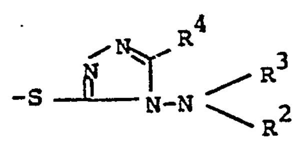

$R^2$ représente H, un reste acétyle, alkyle ayant 1 à 4 atomes de carbone ou $COOR^5$,

$R^3$ est H ou un reste acyle,

$R^4$ est H ou un reste alkyle ayant 1 à 4 atomes de carbone,

$R^5$ est un reste alkyle ayant 1 à 6 atomes de carbone ou cycloalkyle ayant 5 ou 6 atomes de carbone, ou le reste phényle ou benzyle.

3. Matériel suivant la revendication 1, caractérisé en ce que

$R^1$ est un reste méthyle, éthyle, isopropyle, butyle ou cyclohexyle

$R^2$ représente H ou un reste $COOR^5$,

$R^3$ est le reste acétyle ou un reste $COOR^5$

$R^4$ représente H, ou le reste méthyle ou éthyle,

$R^5$ est un reste éthyle, butyle ou cyclohexyle,

4. Matériel suivant la revendication 1, caractérisé en ce que B représente

—O—
—O—$[CH_2]_n$—O— avec n = 1-4
—$[CH_2]_n$— avec n = 1-4

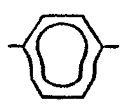 ou 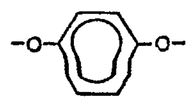

5. Matériel suivant la revendication 1, caractérisé en ce que le composé de formule (I) est présent en une quantité de 5 à 500 mg par mole d'halogénure d'argent.

6. Matériel suivant la revendication 1, caractérisé en ce qu'il renferme au moins l'un des composés suivants

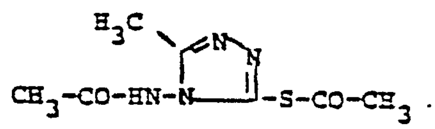

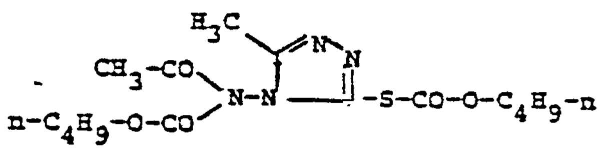

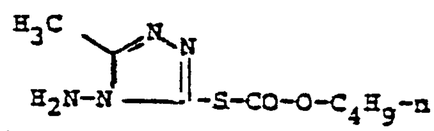

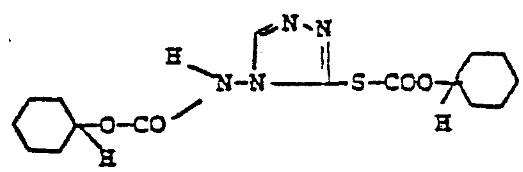

7. Procédé de production de matériels photographiques contenant un halogénure d'argent, par précipitation de l'halogénure d'argent, maturation et application sur une base, caractérisé en ce que l'émulsion d'halogénure d'argent est additionnée d'un composé de formule (I) suivant la revendication 1 avant l'application.

8. Procédé suivant la revendication 7, caractérisé en ce que l'addition est effectuée avant la maturation chimique.

9. Procédé de production d'images photographiques par éclairement au format de l'image et développement d'un support d'enregistrement photographique, caractérisé en ce qu'on utilise un matériel suivant la revendication 1.

10. Composés de formule (I)

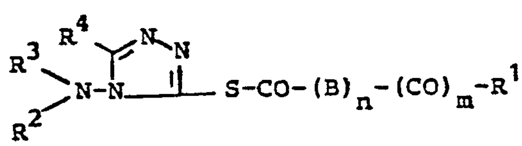 (I)

dans laquelle

$R^1$ désigne un reste alkyle, cycloalkyle, aryle ou aralkyle ou un reste triazole substitué en liaison par l'intermédiaire d'un atome —S—,

$R^2$ représente H, un reste acyle, alkyle ou —$COOR^5$,

$R^3$ représente H, un reste acyle, ou un reste —$COOR^5$,

$R^4$ représente H ou un reste alkyle

$R^5$ est un reste alkyle, cycloalkyle, aryle, aralkyle,

n, m égaux ou différents, ont la valeur 0 ou 1

B représente —O—, un reste alkylène, arylène, cycloalkylène ou aralkylène, ces restes organiques pouvant porter en un ou deux sites un atome d'oxygène,

et les substituants indiqués pouvant quant à eux être substitués avec des substituants qui sont classiques pour des stabilisants dans le domaine de la photographie.